# EUROPEAN PATENT APPLICATION

(11) **EP 1 640 018 A1**
(43) Date of publication of application: **29.03.2006**
(21) Application number: 04022810.8
(22) Date of filing: 24.09.2004
(51) Int. Cl.: A61K 38/20, A61K 38/19, A61K 39/00, A61P 35/00, A61P 33/00, A61P 31/04, A61P 31/12

(54) **Combinational therapy for treating cancer**

(71) Applicant: Universität Zürich, 8006 Zürich (CH)
(72) Inventor: Mölling, Karin, Prof. Dr., 8044 Zürich (CH); Pavlovic, Jovan, PD Dr., 8044 Zürich (CH)
(74) Representative: Grünecker, Kinkeldey, Stockmair & Schwanhäusser Anwaltssozietät

(57) **Abstract**

The present invention relates to means and methods for treating cancer, in particular, the present invention relates to compositions, pharmaceutical compositions comprising the compounds of the compositions suitable for simultaneous, separate or sequential administration, and the use of the compounds of the compositions for preparing a pharmaceutical composition for simultaneous, separate or sequential administration for the prophylaxis or therapy of cancer, neurofibromatosis, and diseases caused by viral, bacterial or parasitic agents.

## Description

The present invention relates to means and methods for treating cancer, in particular, the present invention relates to compositions, pharmaceutical compositions comprising the compounds of the compositions suitable for simultaneous, separate or sequential administration, and the use of the compounds of the compositions for preparing a pharmaceutical composition for simultaneous, separate or sequential administration for the prophylaxis or therapy of cancer, neurofibromatosis, and diseases caused by viral, bacterial or parasitic agents.

Cancer is one of the main causes of death in mankind. Accordingly, there is a lot of ongoing research to develop effective treatments for the prophylaxis/therapy of the various kinds of cancer. Among the molecules under consideration as anti-cancer agents is IL-12.

II-12 is a heterodimeric cytokine of p35 and p40 subunits. IL-12 is produced mainly by monocytes/macrophages. IL-12 is a pleiotropic cytokine. In particular, IL-12 enhances NK- and T-cell-mediated cytotoxicity and stimulates proliferation of these cells, promotes differentiation of Th1 clones and may induce secretion of interferon γ (IFN_{γ}) from both rested and activated NK cells and T lymphocytes. IL-12 has also been demonstrated to play a role in intrathymic T-cell development as well as the maturation of bone marrow cells.

IL-12, when administered *in vivo,* has been shown to exert anti-tumor activity against a number of murine tumors resulting in the inhibition of tumor growth as well as the reduction of the number of metastasis (Rakhmilevich et al., Hum. Gene Ther. 8 (1997), 1303-1311).

Although the aforementioned approach showed some promising reduction in the delay of tumor growth, there remains a need for pharmaceutical compositions with enhanced capability to delay the tumor growth in particular *in vivo.*

The technical problem underlying the present invention is therefore the provision of means and methods exhibiting an enhanced anti-tumor activity.

The technical problem underlying the present invention is solved by the provision of a composition comprising at least two members a) and b) selected from the group consisting of IL-12, CCL19, CCL21, CXCL13, B-Raf kinase inhibitor, a tumor-associated antigen, derivatives or biologically active fragments thereof. Further, a pharmaceutical composition for simultaneous, separate or sequential administration of at least compounds a) and b) as defined above is provided. In addition, the use of at least compounds a) and b) as defined above for preparing a pharmaceutical composition for simultaneous, separate or sequential administration for the prophylaxis or therapy of cancer, neurofibromatosis, diseases caused by viral, bacterial or parasitic agents is provided.

Further solutions are apparent from the subject matter of the claims.

Surprisingly, the present inventors have observed that the above composition when administered as a mixture or separately as single ingredients has an increased anti-tumor activity, in particular with respect to the delay in tumor growth *in vivo* over the administration of a compound alone. Particularly, the observed combined activity was synergistic over the administration of each compound alone. As an example for a combination with a strong synergistic effect, reference is made to the combination of IL-12 with CCL-21, wherein said synergistic effect could not be expected.

Without being bound by theory, the present inventors assumed that by an unknown mechanism the cellular immune response was specifically enhanced over the response observed when only a single compound was applied. Regarding CCL21 and CCL19 both are ligands of the CCR7 receptor wherein CCR7 is expressed on naïve T-cells and dendritic cells.

In the following some terms will be defined to clarify how they should be understood in the context of the present application.

IL-12 is a heterodimer of about 75 kD comprised of two polypeptide subunits, a 40 kD subunit and a 35 kD subunit which are bonded together via one or more disulfide bonds. The human IL-12 polypeptide sequence is shown in Gen bank accession no. AF180563 (p40) and NM000882 (p35) or in Gubler et al. (PNAS USA 88 (1991), 4143-4147). The biological activities of IL-12 comprise the following:
a) activating NK-cells;
b) capability of enhancing the cellular immune mechanisms by directing CD4⁺ T-cells towards a T_{H}1-type response;
c) can stimulate interferon-γ and interleukin-2 secretion;
d) can increase immunogenicity of the tumors by upregulating HLA class I, HLA class II and ICAM-I expression on human melanoma cells;
e) anti-angiogenic property

Preferably, the biological activity of the derivative of the fragment may be determined by measuring the tyrosine phosphorylation of STAT4 in human lymphocytes as described in Bacon et al., PNAS USA 92 (1995), 7307-7311. Alternatively, the anti-tumor activity of IL-12 may be determined in accordance with the examples described below.

"CCL-19" is also known as macrophage inflammatory chemokine-3β (MIP-3β), CK β-11, and Epstein Barr virus-induced ligand chemokine-1 (ELC). The amino acid sequence of CCL-19 is disclosed in Genbank accession no. CAA11307 or in US 6,139,832. The CCL-19 may be of any organism producing such a polypeptide, preferably of mammals, such as mice, most preferably of humans. According to Förster et al., Cell 99 (1999), 23-33, CCL-19 attracts naive T cells and dendritic cells. The biological activity of derivatives or fragments of CCL-19 may be determined by measuring the chemotaxis of T lymphocytes as described in Bardi et al., FEBS Letters 542(2003), 79-83. Alternatively, the anti-tumor activity of CCL-19 may be determined using the *in vivo* assay on the basis of melanoma as described in the examples.

"CCL-21" is also known as CKβ-9, secondary lymphoid tissue chemokine (SLC). 6Ckine, exodus-2; or thymus-derived chemotactic agent-4 (TCA-4). The CCL-21 may be from any organism producing such a polypeptide, preferably of mammals such as mice, most preferably of humans. The human CCL-21 amino acid sequence is disclosed in Genbank accession no. AAQ89246 and US 6,518,046. As CCL-19, CCL-21 also binds to CCR7 and is capable of attracting naive cells T cells and dendritic. The biological activity of derivatives and fragments of CCL-21 may be determined by measuring the chemotaxis of T lymphocytes as described in Bardi et al., supra. Alternatively, the anti-tumor activity of CCL-21, derivatives thereof or fragments may be determined using the *in vivo* melanoma model described in the examples.

"CXCL13" is also known as B-cell attracting chemokine 1/BCA-1 or B-lymphocyte chemoattractant/BLC. The amino acid sequence of CXCL13 is disclosed in Genbank accession No. NP_006410 and Legler et al., J. Exp. Med. 187(1998), 655-660. The CXCL13 may be of any organism producing such a polypeptide, preferably of mammals, such as mice, most preferably of humans. CXCL13 is expressed in lymphoid tissues. The biological activity of derivatives or fragments of CXCL13 may be determined by *in vitro* chemotaxis of B-lymphocytes as described in Legler et al., *supra.*

"B-Raf kinase inhibitor" is generally every compound capable of substantially reducing the serine threonine kinase activity of B-Raf. "Substantially reducing" hereby means a reduction of at least 50%, preferably 80% and most preferably at least 90% of the serine/threonine kinase activity of B-Raf. As an exemplary B-Raf kinase inhibitor, BAY43-9006 described in Karasarides et al., Oncogene 23 (2004), 6292-6298 is indicated. The structural formula of BAY43 - 9006 is shown in Fig. 5A of Wan et al., Cell 116 (855-867). Further, useful B-Raf kinase inhibitors are described in Lackey et al., Bioorg. Med. Chem. Letters 10(2000), 223-226. The biological activity of derivatives or fragments of the B-Raf kinase inhibitor may be determined using the *in vivo* melanoma model used in the examples.

"Tumor-associated antigen" generally means any antigen specific for a tumor or predominantly expressed by a tumor. The antigen is capable of generating a T cell and/or B cell response. Preferably, the tumor-associated antigen is derived from melanoma, stomach cancer, colon cancer, pancreas carcinoma, small cell lung cancer, lung cancer, liver carcinoma, prostate cancer, gall bladder cancer, squamous cell carcinoma, mammary carcinoma, testes cancer, ovarial carcinoma, B cell lymphoma, angiosarcoma, fibrosarcoma, neurofibroma and malignant peripheral nerve sheath tumor. Particularly preferred the tumor-associated antigen is selected from the group consisting of gp100, tyrosinase, MAGE-1, MAGE-3, MART, BAGE, TRP-1, carcinoma embryonic antigen (CEA), CA19-9, CA50, CA72-4 Muc-1, neuron-specific enolase (NSE), EGF receptor, alpha-fetoprotein, PSA, squamous cell carcinoma antigen (SSC), CA15-3, BRCA-1, BRCA-2, Her2/Neu receptor, AFP, hCG, CA-125, AFP, TAG-72, CD20, CD21, or an immunoreactive fragment thereof. The amino acid and nucleic acid sequences of the afore-mentioned tumor-associated antigens are available from public databases such as Genbank or SwissProt™.

An "immunoreactive fragment" is a fragment of a tumor-associated antigen being capable of generating a T cell and/or B cell response when administered to a human or an animal, preferably a mammal. The immunoreactive fragment preferably comprises at least 10, more preferably at least 20 and most preferably at least 50 amino acid residues.

The immunoreactivity of the derivative or fragment of the tumor-associated antigen may be determined using melanoma-reactive cytotoxic T cells as described in Salgaller et al., Cancer Res. 55(1995), 4972-4979). As a preferred example for a tumor-associated antigen, gp100 is the preferred antigen. The gp100 polypeptide may be of any organism producing such a polypeptide, preferably of mammals, such as mice, most preferably of humans. The amino acid sequence and nucleic acid sequence of GP100, preferably human, GP100 is known. It is disclosed in Genbank accession no. AAC60634.

"Oligopeptide" means a peptide having up to 100 amino acids. "A polypeptide" means a peptide having at least 100 amino acids.

A "nucleic acid capable of encoding a compound" refers to any possible nucleic acid molecule encoding the oligopeptide or polypeptide form of the compound. Thus, the term includes RNA or DNA or mixed forms thereof, linear or circular, single-stranded or double-stranded, modified or unmodified. Preferably, the nucleic acid is DNA. Further preferred, the DNA is a double-stranded DNA, more preferably a circular one and even more preferably a plasmid or a vector. The term "nucleic acid" also includes unpackaged DNA. "Unpackaged DNA" means DNA in an aqueous solution without viral vectors. The DNA solution may contain stabilizers or agents for protection against nucleases. Particularly preferred, the nucleic acid is naked DNA. "Naked" in this regard preferably means that the DNA is not complexed to viral structures, proteins or lyposomes or the like, most preferably the DNA is present in fluid medium which does not contain any components apart from water and a buffer.

"Percent identity" means the percent identity relative to the unaltered sequence. For example, an 80% identity means that for a 100 amino acid comprising sequence that up to 20 of the 100 amino acids may differ from the unaltered sequence.

The percent identity may be determined using standard programs in the art such as BLAST or FASTA, preferably the standard default parameters are used.

The term "derivative" comprises any modification of a given amino acid or nucleic acid sequence. Preferably, the derivative is a mutein differing from the unaltered sequence by the addition, substitution, deletion, insertion or inversion of at least one amino acid residue or one nucleotide, respectively, from the unaltered sequence. More preferably, the mutein differs from the unaltered sequence by not more than five amino acids or 15 nucleotides, respectively.

The composition according to the present invention comprises:
a) IL-12, a derivative or biologically active fragment thereof; and
b) at least one member selected from the group consisting of CCL19, CCL21, CXCL13, B-Raf kinase inhibitor, a tumor-associated antigen, derivatives or biologically active fragments thereof.

In a further preferred embodiment, the composition comprises:
a) CCL19, a derivative or biological active fragment thereof; and
b) at least one member selected from the group consisting of IL-12, CCL21, CXCL13, B-Raf kinase inhibitor, a tumor-associated antigen, derivatives or biologically active fragments thereof.

According to a further preferred embodiment, the composition comprises
a) CCL21, a derivative or biologically active fragment thereof;
b) At least one member selected from the group consisting of IL-12, CCL19, CXCL13, B-Raf kinase inhibitor, a tumor-associated antigen, derivatives or biologically active fragments thereof.

Particularly preferred, the composition comprises IL-12 and CCL19 and/or CCL21. Most preferred, the composition comprises IL-12 and CCL21.

In a further preferred embodiment, the composition comprises
a) CXCL13, a derivative or biologically active fragment thereof; and
b) At least one member selected from the group consisting of IL-12, CCL19, CCL21, B-Raf kinase inhibitor, a tumor-associated antigen, derivatives or biologically active fragments thereof.

In another preferred embodiment the composition comprises:
a) B-Raf kinase inhibitor, a derivative or biologically active fragment thereof; and
b) at least one member selected from the group consisting of IL-12, CCL19, CCL21, CXCL13, a tumor-associated antigen, derivatives or biologically active fragments thereof.

In another preferred embodiment the composition comprises:
a) a tumor-associated antigen, a derivative or biologically active fragment thereof; and
b) at least one member selected from the group consisting of IL-12, CCL19, CCL21, CXCL13, B-Raf kinase inhibitor, derivatives or biologically active fragments thereof.

It is further preferred that at least compounds a) and b) are both oligo- or polypeptides. Alternatively, it is preferred that compound a) is a nucleic acid capable of encoding compound a), and compound b) is an oligo- or polypeptide; or *vice versa,* i.e. that compound a) is an oligo- or polypeptide, and compound b) is a nucleic acid capable of encoding compound b). According to a further preferred embodiment, the compounds a) and b) in the composition are nucleic acids capable of encoding compounds a) and b), respectively.

If compounds a) and b) are oligo- or polypeptides, they may be produced synthetically by e.g. Merrifield synthesis or fragment condensation or by recombinant expression in an appropriate host cell system. Appropriate recombinant expression systems are known in the art. Suitable host organisms may e.g. be *E*. *coli, Saccharomyces cerevisiae,* HELA cells, CHO or COS cells. Appropriate vector systems may e.g. be plasmids or baculovirus expression vectors.

If the compound a) and/or b) is a nucleic acid the nucleic acid may either be obtained by cloning or PCR from natural sources or be produced synthetically.

The derivative as mentioned in the composition outlined above may preferably be a mutein differing from the unaltered sequence by the addition, deletion, substitution, inversion or insertion of at least one amino acid, preferably of not more than five amino acids. If the unaltered sequence is a nucleic acid sequence, then the derivative is a mutein differing therefrom by the addition or deletion, substitution, inversion or insertion of at least one nucleotide, preferably of not more than 15 nucleotides, therefrom.

The derivative is preferably at least 80%, more preferably 90%, most preferably 95% identical to the unaltered sequence.

The derivative and/or biologically active fragment of compounds a) and/or b) as mentioned above exhibits preferably at least 80%, more preferably 90%, and most preferably 95% of the biological activity of the unaltered sequence. The biological activity of the unaltered sequence with respect to the compounds indicated in the claims may be determined as outlined above.

According to another aspect of the present invention, a method for preparing the composition is provided comprising the step of mixing compounds a) and b) and optionally, further members of the groups as indicated above.

When preparing the composition suitable stabilizers or additives may be added. Suitable stabilizers and additives are well-known in the art.

According to another aspect of the present invention, a pharmaceutical composition for simultaneous, separate or sequential administration of at least compounds a) and, b) as defined above is provided.

It is noted that the pharmaceutical composition is not limited to a pharmaceutical composition comprising both compounds a) and b) in a mixture, but the term "a pharmaceutical composition for simultaneous, separate or sequential administration" also includes the possibility that compounds a) and b) are physically separate from each other before administration but are intended to be administered to a human or animal, in particular, to a patient. The scope of the claims therefor are encompass the possibility that compounds a) and b) are together or separate before administration to the animal or human. The pharmaceutical composition may further comprise pharmaceutically acceptable carriers or adjuvants well-known in the art of preparing pharmaceuticals.

The pharmaceutical composition may preferably further comprise a chemotherapeutic agent. The chemotherapeutic agent may depend on the disease to be treated. Suitable chemotherapeutics are known to the physician. Preferably, the pharmaceutical composition is suitable for oral, intravenous, intramuscular, subcutaneous, intradermal or intratumoral administration, wherein intratumoral administration is preferred. If the pharmaceutical composition is for separate or sequential administration of compounds a) and b), then the two compounds a) and b) may be formulated differently from each other. For example, compound a) is suitable for oral administration and compound b) is formulated for intratumoral administration.

In the context of the present invention, "intratumoral administration" means that the compound a) and/or b) is delivered directly into the tumor, i.e. into actively dividing tumor cells surrounding the necrotic central part of the tumor and not e.g. only into peritumoral cells or into the center of the tumor.

The term "tumor" in this context does not only refer to the primary tumor but also to metastasis. Appropriate means for intratumoral administration are e.g. injection, ballistic tools, electroporation electroinsertion, wounding, scratching, pressurized insertion tools, dermojets, etc.

In a preferred embodiment, the intratumoral administration is carried out by injection, preferably by a needle and a syringe. A suitable solution in this regard is e.g. phosphate buffered saline (PBS), citrate buffer, Tris-HCl buffer or any physiological buffer.

The present invention provides the use of at least compounds a) and b) as defined above for preparing a pharmaceutical composition for simultaneous, separate or sequential administration for the prophylaxis or therapy of cancer, neurofibromatosis, or diseases caused by viral, bacterial or parasitic agents.

Cancer hereby includes melanoma, stomach cancer, colon cancer, pancreas carcinoma, small cell lung cancer, lung cancer, liver carcinoma, prostate cancer, gall bladder cancer, squamous cell carcinoma, mammary carcinoma, testes cancer, ovarial carcinoma, B cell lymphoma, angiosarcoma, fibrosarcoma, neurofibroma and malignant peripheral nerve sheath tumor. Particularly preferred is melanoma. Further examples for tumors are head and neck adeno carcinoma, renal cell, etc. and sarcomas (fibro-, bone, Ewing, Kaposi, etc.) and mastocytoma, basalioma, haemangioma, etc.

If the cancer to be treated is malignant melanoma and the pharmaceutical composition suitable for simultaneous, separate or sequential administration comprises a tumor-associated antigen, then the tumor-associated antigen may be MAGE, MART, gp100 or tyrosinase; see also Rosenberg et al., Immunol. Today 18 (1997), 175-182.

If the pharmaceutical composition suitable for simultaneous, separate or sequential administration comprises IL-12, it is preferred that IL-12 is administered as a nucleic acid molecule encoding IL-12 and the further member of the groups as indicated above are applied as oligo- or polypeptide. The intratumoral administration of IL-12 has been shown to be very effective; see Rakhmilevich et al., Hum. Gene Ther. 8 (1997), 1303-1311.

The diseases caused by viral, bacterial or parasitic agents may be selected from Leishmania, Trypanosoma cruzi, Toxoplasma gondii, Plasmodium falciparum, Mycobacterium tuberculosis, Cryptococcus neoformans, Tropheryma whipplei.

If compound a) and/or b) is an oligo- or polypeptide, suitable concentration ranges are from 0.5 µg to about 1 mg.

If compound a) and/or b) is a nucleic acid molecule, then the concentration ranges from 50 µg to about 1 mg.

Preferably, the composition is suitable for intratumoral administration of compound a) or b).

The present invention further relates to a method for the prophylaxis or therapy of cancer, neurofibromatosis, diseases caused by viral, bacterial or parasitic agents, comprising administering at least compounds a) and b) as defined above to a patient in need thereof. Preferably, compound a) and/or b) is administered more than once. In particular, it can be advantageous if compound a) and/or b) is a nucleic acid molecule to administer the nucleic acid molecule by intratumoral administration repeatedly, e.g. at least twice or three times on day 1, 3 and/or 5. When administration is carried out by injection, more than one injection is preferred (e.g. 5 to 6 injections). Furthermore, the needle is preferably inserted tangential to the tumor and is not pointing to the center of the tumor. An example for a therapy protocol is 50 µg of DNA as a pre-dose seven days before a triple dose, i.e. three doses a day 1, 3, 5, or 1, 8, 15. The triple dose can be repeated after two or three weeks. It can also be repeated several times (3 or 4 times) and repeated when the tumor occurs again. Thus, the treatment may consist of 1 or 2 or more cycles.

It is particularly preferred that a pre-dose of the nucleic acid molecule is administered about 14 days before the actual treatment starts. Preferably, the pre-dose comprises about 50 µg of naked plasmid DNA. Such a pre-dose prevents toxic side effects of the administered compound (Leonard et al., Blood 90 (1997), 2541-2548). A particularly preferred embodiment for the mode of administration is a regimen in which the administration of the pre-dose is followed by two cycles of administration and wherein in cycle 1, 100 µg to 750 µg of naked plasma DNA are administered on day 1, 8 and 15 and wherein in cycle 2, 200 µg to 1000 µg of naked plasma DNA are administered on days 23, 36 and 43.

The references as cited herein are incorporated herewith by reference. The following examples are intended to illustrate the invention but do not limit the scope of the invention.

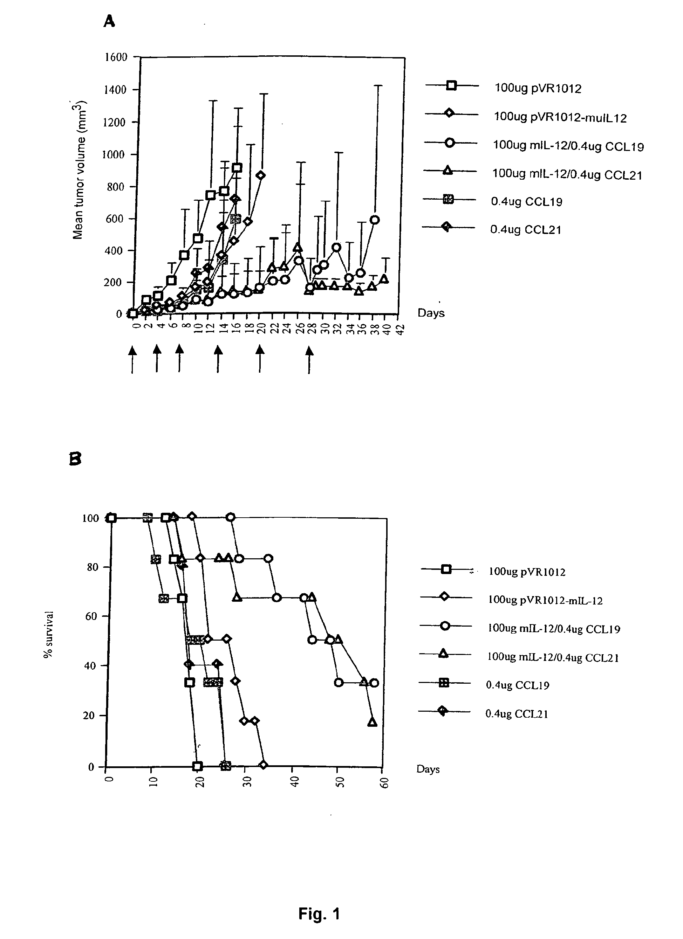

Fig 1. *Therapeutic effect of IL-12 DNA in combination with chemokines.* To evaluate the effect of local treatment with IL-12 DNA in combination with CCL21 and CCL19 on tumor growth we used the melanoma-bearing C57BL/6 mice. Tumors were established by subcutaneous inoculation of 1×10⁵ B16F10 melanoma cells into the right hind flank. When a mean tumor volume of 3x3-6x6 mm² was reached (day 0), treatment was started. Groups of 6 mice were intratumorally injected with 100ug pVR1012-mulL-12, alone or in combination with either 0.4ug CCL19 or CCL21 or as controls 100ug pVR1012, 0.4 ug CCL19 or CCL21 on days 0, 2, 4, 8, 14, 20, and 28. A) Growth kinetics B) percentage of survival of mice treated or untreated with chemokines. Mean tumor volumes of groups ± standard deviations are depicted

### Materials and Methods

### Plasmid DNA

Cloning and large scale production of the plasmid DNA encoding murine IL-12 (pVR1012-mulL-12) has been described previously (Schultz et al. Hum. Gene Ther. 10 (1999), 407-417). Recombinant murine exodus-2/CCL21 and recombinant murine MIP-3β/CCL19 were purchased from PeproTech, London, UK.

### In vivo experiment

Female C57BL/6 mice, 6-8 weeks of age were bred in the Institute of Medical Virology, Zurich, Switzerland. To establish local melanoma, 1x10⁵ B16F10 melanoma cells were subcutaneousely inoculated in the right flank of mice. As soon as the tumors reached a size of approximately 20 mm³ (day 0), 100 µg of plasmid DNA and/or 0.4 µg of the chemokines were injected for the first time. Repeated inoculations were performed at the indicated intervals for a total of 6 applications. Tumor volumes were determined by caliper every second day. The tumor volumes was estimated by the formula: 1/2(length x width²).
Statistical differences between groups were determined by the Student's *t*-test. P<0.05 was considered as statistically significant.

### Results

As shown in Fig.1, tumors progressed more slowly in treated mice with IL-12 DNA, recombinant CCL21, and recombinant CCL19, compared to control mice injected with only PBS. Recombinant chemokines, CCL21 and CCL19 exert a similar antitumor effect as DNA IL-12. The combination therapy of DNA IL-12 with CCL21 or CCL19 showed a greater tumor growth retardation. However the strongest therapeutic effect was observed in mice treated with IL-12 DNA in combination with CCL21 (P<0.05).

## Claims

1. A composition comprising at least two members a) and b) selected from the group consisting of IL-12, CCL19, CCL21, CXCL13, B-Raf kinase inhibitor, a tumor-associated antigen, derivatives or biologically active fragments thereof.

2. The composition according to claim 1 wherein the composition comprises:
a) IL-12, a derivative or biologically active fragment thereof; and
b) at least one member selected from the group consisting of CCL19, CCL21, CXCL13, B-Raf kinase inhibitor, a tumor-associated antigen, derivatives or biologically active fragments thereof.

3. The composition according to claim 1 wherein the composition comprises:
a) CCL19, a derivative or biologically active fragment thereof; and
b) at least one member selected from the group consisting of IL-12, CCL21, CXCL13, B-Raf kinase inhibitor, a tumor-associated antigen, derivatives or biologically active fragments thereof.

4. The composition according to claim 1 wherein the composition comprises:
a) CCL21, a derivative or biologically active fragment thereof; and
b) at least one member selected from the group consisting of IL-12, CCL19, CXCL13, B-Raf kinase inhibitor, a tumor-associated antigen, derivatives or biologically active fragments thereof.

5. The composition according to any of claims 2 to 4 wherein the composition comprises:
a) IL-12; and
b) CCL19 and/or CCL21.

6. The composition according to claim 1 wherein the composition comprises:
a) CXCL13, a derivative or biologically active fragment thereof; and
b) at least one member selected from the group consisting of IL-12, CCL19, CCL21, B-Raf kinase inhibitor, a tumor-associated antigen, derivatives or biologically active fragments thereof.

7. The composition according to claim 1 wherein the composition comprises:
a) B-Raf kinase inhibitor, a derivative or biologically active fragment thereof; and
b) at least one member selected from the group consisting of IL-12, CCL19, CCL21, CXCL13, a tumor-associated antigen, derivatives or biologically active fragments thereof.

8. The composition according to claim 1 wherein the composition comprises:
a) a tumor-associated antigen, a derivative or biologically active fragment thereof; and
b) at least one member selected from the group consisting of IL-12, CCL19, CCL21, CXCL13, B-Raf kinase inhibitor, derivatives or biologically active fragments thereof.

9. The composition according to any of claims 1 to 8 wherein the tumor-associated antigen is derived from melanoma, stomach cancer, colon cancer, pancreas carcinoma, small cell lung cancer, lung cancer, liver carcinoma, prostate cancer, gall bladder cancer, squamous cell carcinoma, mammary carcinoma, testes cancer, ovarial carcinoma, B cell lymphoma, angiosarcoma, fibrosarcoma, neurofibroma or malignant peripheral nerve sheath tumor.

10. The composition according to claim 9 wherein the tumor-associated antigen is selected from the group consisting of gp100, tyrosinase, MAGE-1, MAGE-3, MART, BAGE, TRP-1, carcino embryonic antigen (CEA), CA 19-9, CA 50, CA 72-4, Muc-1, neuron specific enolase (NSE), EGF receptor, alpha-fetoprotein, PSA, squamous cell carcinoma antigen (SSC), CA 15-3, BRCA-1, BRCA-2, Her2/Neu receptor, AFP, hCG, CA-125, AFP, TAG-72, CD20, CD21, or an immunoreactive fragment thereof.

11. The composition according to claim 10 wherein the immunoreactive fragment comprises at least 10, preferably at least 20, and most preferably at least 50 amino acids.

12. The composition according to any of claims 1 to 11 wherein at least compounds a) and b) are both oligo- or polypeptides.

13. The composition according to any of claims 1 to 11 wherein compound a) is a nucleic acid capable of encoding compound a) and compound b) is an oligo- or polypeptide; or compound a) is an oligo- or polypeptide and compound b) is a nucleic acid capable of encoding compound b).

14. The composition according to any of claims 1 to 11 wherein compounds a) and b) are nucleic acids capable of encoding compounds a) and b), respectively.

15. The composition according to any of claims 1 to 14 wherein the derivative is a mutein differing from the unaltered sequence by the addition, deletion, substitution, insertion or inversion of at least one amino acid, preferably not more than 5 amino acids, therefrom.

16. The composition according to any of claims 1 to 15 wherein the derivative is at least 80 %, preferably 90 %, most preferably 95 % homologous to the unaltered sequence.

17. The composition according to any of claims 1 to 16 wherein derivative and/or the biologically active fragment exhibits at least 80 %, preferably 90 %, most preferably 95 % of the biological activity of the unaltered sequence.

18. A method for preparing a composition of any of claims 1 to 17 comprising the step of mixing compounds a) and b) and, optionally, further members of the group.

19. A pharmaceutical composition comprising CCL19, a derivative or biologically active fragment thereof.

20. A pharmaceutical composition comprising CCL21, a derivative or biologically active fragment thereof.

21. A pharmaceutical composition comprising CXCL13, a derivative or biologically active fragment thereof.

22. A pharmaceutical composition for simultaneous, separate or sequential administration of at least compounds a) and b) as defined in any of claims 1 to 17.

23. The pharmaceutical composition of any of claims 19 to 22 further comprising a chemotherapeutic agent.

24. The pharmaceutical composition of any of claims 19 to 23 wherein the composition is suitable for oral, intravenous, intramuscular, subcutaneous, intradermal or intratumoral administration, preferably intratumoral administration.

25. Use of at least compounds a) and b) as defined in any of claims 1 to 17 for preparing a pharmaceutical composition for simultaneous, separate or sequential administration for the prophylaxis or therapy of cancer, neurofibromatosis, or diseases caused by viral, bacterial or parasitic agents.

26. Use of claim 25, wherein the composition is suitable for intratumoral administration of compound a) and/or b).

27. A method for the prophylaxis or therapy of cancer, neurofibromatosis, or diseases caused by viral, bacterial or parasitic agents comprising administering at least compounds a) and b) as defined in any of claims 1 to 17 to a patient in need thereof.

28. The method of claim 27 wherein compound a) and/or compound b) is/are intratumorally administered.
